# EUROPÄISCHE PATENTSCHRIFT

(11) **EP 3 110 268 B1**
(45) Veröffentlichungstag und Bekanntmachung des Hinweises auf die Patenterteilung: **25.04.2018**
(21) Anmeldenummer: 15708475.7
(22) Anmeldetag: 27.02.2015
(51) Int. Cl.: A24F 47/00

(54) **RAUCHVORRICHTUNG**
SMOKING DEVICE
DISPOSITIF FUMOIR

(30) Priorität: 27.02.2014 DE 202014001718 U; 01.10.2014 DE 102014114308
(43) Veröffentlichungstag der Anmeldung: 04.01.2017
(73) Patentinhaber: Xeo Holding GmbH, 30167 Hannover (DE)
(72) Erfinder: GOCH, Markus, 30167 Hannover (DE)
(74) Vertreter: Gottschald, Jan
(86) Internationale Anmeldenummer: PCT/EP2015/054217
(87) Internationale Veröffentlichungsnummer: WO 2015/128499

(56) Entgegenhaltungen:
- EP-A1- 2 481 308
- WO-A1-2010/107613
- US-A1- 2013 014 772

## Beschreibung

Die Erfindung betrifft eine Rauchvorrichtung gemäß dem Oberbegriff von Anspruch 1.

Als Alternative zu herkömmlichen Zigaretten, Zigarren, Pfeifen und anderen Vorrichtungen oder Verbrauchsartikeln zur Tabakverbrennung sind aus dem Stand der Technik verschiedene Rauchvorrichtungen bekannt.

Hierzu zählen insbesondere elektronische Zigaretten. Diese funktionieren regelmäßig nach dem Prinzip eines Liquidverdampfers, gemäß dem ein zu verdampfendes Liquid, welches mit Nikotin oder anderen Geschmacks- oder Wirkstoffen versetzt sein kann, aus einem Reservoir mittels eines Dochtes zu einer elektrisch gespeisten Heizspirale gelangt und dort verdampft wird. Dieses Funktionsprinzip erlaubt es, innerhalb einer sehr kurzen Ansprechzeit verhältnismäßig große Mengen an Liquid zu einem Aerosol zu verdampfen und dieses einen an der elektronischen Zigarette ziehenden Benutzer einatmen zu lassen. Das Verfahren erlaubt es auch, die Menge an Liquiddampf an die Zugintensität des Benutzers an der elektronischen Zigarette zu koppeln.

Aus dem Stand der Technik sind außerdem elektrische Tabakerhitzer - welche auch als "Tobacco Heater" bezeichnet werden - bekannt, bei denen nach zunächst erfolgender Erhitzung auf eine Vorheiztemperatur eine Tabaksubstanz auf eine Verfahrenstemperatur weiter erhitzt wird, bei welcher Verfahrenstemperatur die Tabaksubstanz einen Tabakdampf abgibt, ohne dass es zu einer wesentlichen Verbrennung des Tabaks wie bei einer herkömmlichen Zigarette kommt. Das führt dazu, dass der durch den Tabakerhitzer entstehende Tabakdampf das angenehme Aroma und die warme Temperatur von herkömmlichem Zigarettenrauch aufweist, aber frei von den wesentlichen schädlichen Bestandteilen dieses Rauchs ist, die eine Folge des Verbrennungsprozesses sind.

Nachteilig an den obigen elektronischen Zigaretten ist, dass der von ihnen erzeugte Dampf eine geringere Temperatur als herkömmlicher Tabakrauch aufweist und ihm ebenso das warme Aroma von Tabak fehlt. Nachteilig an den aus dem Stand der Technik bekannten Tabakerhitzern ist andererseits, dass sie nur eine geringe Dampfmenge erzeugen können und diese Dampfmenge auch nicht ohne Weiteres durch ein Saugen des Benutzers an einem Mundstück einstellbar ist. Hinzu kommt, dass die Tabakerhitzer auf eine Vorheiztemperatur vorerhitzt werden müssen und dieser Vorgang des Erhitzens bis zu dreißig Sekunden dauern kann, was insbesondere gegenüber herkömmlichen Zigaretten einen bedeutenden Verlust an Benutzerkomfort bedeutet.

Um nun Benutzern einen Dampf zu bieten, welcher Nikotin und einen Tabakgeschmack, aber keine Schadstoffe aus der Tabakverbrennung aufweist, schlägt die amerikanische Offenlegungsschrift US 2013/0014772 A1, von welcher die vorliegende Erfindung ausgeht, eine Rauchvorrichtung vor, bei der eine nach dem obigen Verdampferprinzip verdampfte Extraktionssubstanz durch eine Heizkammer eines Tabakerhitzers geführt wird, um dort das Nikotin aus dem Tabak in den Dampf aufzunehmen und an ein Mundstück weiterzuführen, an dem der Benutzer diesen Dampf dann einatmen kann.

Dabei ist die aus diesem Stand der Technik bekannte Vorrichtung allerdings insoweit nachteilig, als dass der Dampf mit der Extraktionssubstanz durch die Heizkammer der Länge nach vollständig durchgeführt wird. Denn um zuverlässig ein warmes Tabakaroma und einen entsprechenden Dampf zu erreichen, ist Trockenheit innerhalb der Heizkammer vorteilhaft. Durch die Anordnung gemäß diesem Stand der Technik sammelt sich allerdings notwendigerweise das Liquid und die entsprechende Feuchtigkeit in der Heizkammer während des Einatmens des Dampfes an. Auf diese Weise wird die Art der Dampferzeugung in der Heizkammer, wie sie eigentlich gewünscht wäre, durch das Durchströmen des verdampften Liquids verhindert. Deshalb können die Vorteile der beiden grundsätzlichen Dampferzeugungsansätze eben nicht wirksam kombiniert werden.

Vor diesem Hintergrund besteht die Aufgabe der Erfindung darin, eine solche Rauchvorrichtung, welche grundsätzlich die Funktionalität eines Liquidverdampfers und eines Tabakerhitzers kombiniert, so weiterzuentwickeln, dass die jeweiligen Vorteile der einzelnen Verfahren sich wirksam ergänzen können.

Das genannte Problem wird bei einer Rauchvorrichtung mit den Merkmalen des Oberbegriffs von Anspruch 1 durch die Merkmale des kennzeichnenden Teils von Anspruch 1 gelöst.

Wesentlich für die Erfindung ist die Erkenntnis, dass der Strömungskanal, durch welchen der Liquiddampf von dem Liquidverdampfer zu einer Ansaugöffnung der Rauchvorrichtung strömt, außerhalb eines Innenraums der Heizkammer, in welcher die Tabaksubstanz erhitzt wird, verläuft. Auf diese Weise durchströmt der Liquiddampf also nicht die Heizkammer und führt nicht zu einer Beeinträchtigung bei der Erzeugung des Tabakdampfs. Damit wird eine Kombination dieser beiden Dampferzeugungsverfahren geboten, welche ihre jeweiligen Vorteile kombiniert. Durch den Liquiddampf wird das - einfach steuerbare - Volumen beim Einatmen bereitgestellt, während der Tabakerhitzer das Tabakaroma und die Temperatur liefert. Es ist erkannt worden, dass bereits ein geringes Volumen an Tabakdampf aus dem Tabakerhitzer ausreicht, um das Aroma und die Temperaturempfindung des Liquiddampfes deutlich zu beeinflussen.

Dieses Verlaufen des Strömungskanals für den Liquiddampf, welcher auch als Liquidströmungskanal bezeichnet werden kann, außerhalb des Heizkammerinnenraums kann einerseits dadurch erfolgen, dass im Gegensatz zum nächstkommenden Stand der Technik, in Strömungsrichtung nicht der Tabakerhitzer auf den Liquidverdampfer folgt, sondern vielmehr der Tabakerhitzer - von dem Liquidverdampfer aus gesehen - stromaufwärts angeordnet ist. In so einem Fall kann dann der Tabakdampf etwa bypassartig an dem Liquidverdampfer vorbei geführt werden, sodass dann stromabwärts von dem Liquidverdampfer ein Zusammenführen der beiden Strömungskanäle erfolgt.

Es kann aber auch der Tabakdampf durch den Liquidverdampfer selber geführt werden. Diese Variante ist in der bevorzugten Ausführung gemäß dem Anspruch 2 beschrieben.

Alternativ kann es aber auch so sein, dass der Liquidströmungskanal an der Heizkammer vorbei geführt wird und mit dem Strömungskanal für den Tabakdampf - hier als Tabakströmungskanal bezeichnet - erst zusammengeführt wird, nachdem der Tabakdampf die Kammer bereits verlassen hat. Der Liquidströmungskanal bildet also eine Art "Bypass" der Heizkammer. Diese Alternative wird in einer bevorzugten Ausführung gemäß dem Unteranspruch 3 beschrieben.

Bevorzugt ist für den Liquidverdampfer und für den Tabakerhitzer jeweils ein eigenes Modul vorgesehen. Diese Module können auch in jeweils eigenen Gehäuseteilen angeordnet sein, die wiederum lösbar miteinander verbunden sind, was Gegenstand des bevorzugten Unteranspruchs 4 ist. Auf diese Weise kann auch die Konfiguration bezüglich einer Reihenfolge der Module im Hinblick auf die Strömungsrichtung zwischen dem Tabakerhitzermodul und dem Liquidverdampfermodul variiert werden.

Dabei stellen Bajonettverbindungen zwischen den einzelnen Modulen besonders bevorzugte Möglichkeiten zum Herstellen einer Verbindung dar, wie durch den Unteranspruch 5 beschrieben wird.

Gemäß dem Unteranspruch 6 können neben dem Liquidverdampfermodul und dem Tabakerhitzermodul auch noch ein Akkumodul sowie sonstige Module vorgesehen sein, wobei das Akkumodul die elektrische Energie sowohl für das Tabakerhitzermodul als auch für das Liquidverdampfermodul bereitstellen kann.

Vorteile ergeben sich weiter, wenn die Verfahrenstemperatur des Tabakerhitzers, bei welcher Temperatur der Tabakdampf aus der Tabaksubstanz entsteht, einstellbar und insbesondere durch eine Tastvorrichtung einstellbar ist. Dies ist in Unteranspruch 8 beschrieben.

Die grundsätzliche Lösbarkeit zwischen dem Liquidverdampfermodul und dem Tabakerhitzermodul kann vorteilhafterweise dazu eingesetzt werden, dass durch ein Ablösen des Liquidverdampfermoduls von dem Tabakerhitzermodul ein Zugang zu der Heizkammer geschaffen wird, in welcher die zu verdampfende Tabaksubstanz eingebracht werden kann. Dies ist Gegenstand des Unteranspruchs 9.

Vorteilhafte Ausgestaltungen des Liquidströmungskanals und des Tabakströmungskanals sowie des Verlaufs des Luftstroms von einer Einlassöffnungsanordnung werden in den Unteransprüchen 10 bis 13 beschrieben.

Dabei sieht eine Variante gemäß dem Unteranspruch 11 vor, dass der eintretende Luftstrom - anstatt lediglich an einem Auslass der Heizkammer vorbeizuziehen - zur intensiveren Aufnahme des Tabakdampfs durch die Heizkammer hindurch verläuft. Der Unteranspruch 14 sieht eine Weiterentwicklung dieser Variante dadurch vor, dass eine Separieranordnung einen Kanal für diesen Luftverlauf von der Tabaksubstanz trennt.

Weitere Einzelheiten, Merkmale, Ziele und Vorteile der vorliegenden Erfindung werden nachfolgend anhand einer lediglich Ausführungsbeispiele wiedergebenden Zeichnung näher erläutert. In der Zeichnung zeigt
- Fig. 1: eine Seitenansicht eines Längsschnitts einer vorschlagsgemäßen Rauchvorrichtung gemäß einem ersten Ausführungsbeispiel,
- Fig. 2: eine vergrößerte Ansicht im Längsschnitt des Liquidverdampfers der vorschlagsgemäßen Rauchvorrichtung der Fig. 1,
- Fig. 3: eine vergrößerte Ansicht im Längsschnitt des Tabakerhitzers der vorschlagsgemäßen Rauchvorrichtung der Fig. 1,
- Fig. 4: eine Seitenansicht eines Längsschnitts einer vorschlagsgemäßen Rauchvorrichtung gemäß einem zweiten Ausführungsbeispiel,
- Fig. 5: eine Seitenansicht eines Längsschnitts einer vorschlagsgemäßen Rauchvorrichtung gemäß einem dritten Ausführungsbeispiel und
- Fig. 6: eine Seitenansicht eines Längsschnitts einer vorschlagsgemäßen Rauchvorrichtung gemäß einem vierten Ausführungsbeispiel.

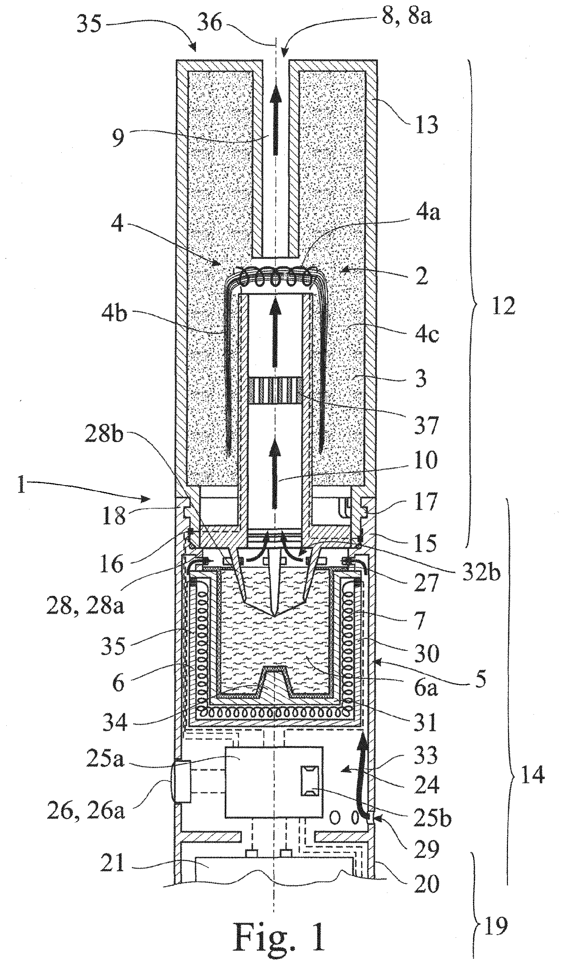

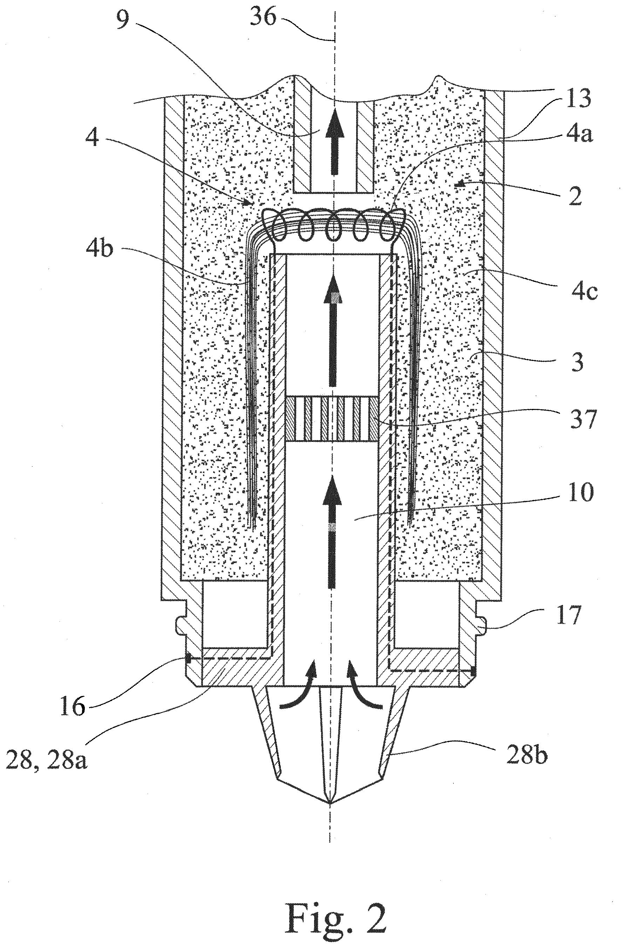

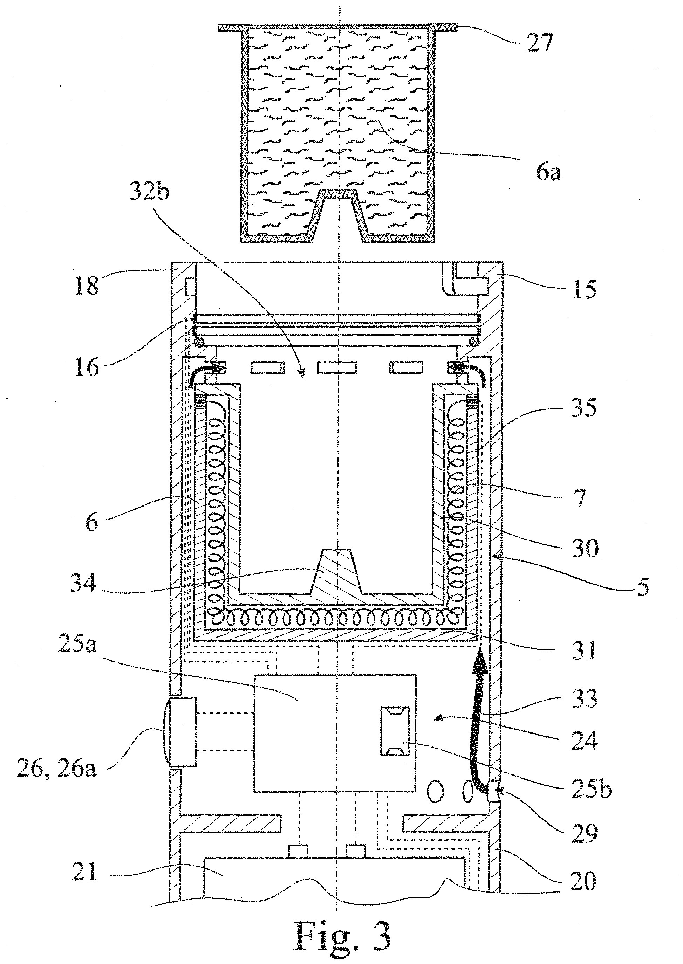

Für die in den Figuren 1, 2, 3 und 4 dargestellten vorschlagsgemäßen Rauchvorrichtungen gilt, dass sie jeweils ein Gehäuse 1 und einen in dem Gehäuse 1 angeordneten Liquidverdampfer 2 aufweisen. Dieser Liquidverdampfer 2 weist seinerseits ein Reservoir 3 für Liquid und eine Liquidheizvorrichtung 4 zum Verdampfen des Liquids in Liquiddampf auf. Die Liquidheizvorrichtung 4 funktioniert dabei nach einem an sich aus dem Stand der Technik bekannten Prinzip, bei dem nämlich eine Heizspule 4a elektrisch erhitzt wird und mittels eines Dochts 4b das Liquid aus dem Reservoir 3, welches z. B. ein mit Liquid getränktes Vlies 4c aufweisen kann, zur Heizspule 4a transportiert wird, wo das Liquid dann verdampft wird.

Die Rauchvorrichtung 1 weist vorschlagsgemäß weiter einen in dem Gehäuse 1 angeordneten Tabakerhitzer 5 auf, wobei dieser Tabakerhitzer 5 eine Heizkammer 6 zur Aufnahme einer Tabaksubstanz in einem Kammerinnenraum 6a und einer Kammerheizvorrichtung 7 zum Erzeugen von Tabakdampf aus der Tabaksubstanz aufweist. Auch die Funktionsweise dieser Kammerheizvorrichtung 7 zum Erzeugen von Tabakdampf ist an sich aus dem Stand der Technik bekannt und wird dort auch als "Tobacco Heating" oder "Toasting" bezeichnet.

Die vorschlagsgemäße Rauchvorrichtung weist ebenso eine Ansaugöffnungsanordnung 8 zum Ansaugen des Liquiddampfes und zum Ansaugen des Tabakdampfs aus dem Gehäuse 1 auf. Insbesondere kann es sich dabei um ein oder mehrere Ansaugöffnungen handeln. Vorschlagsgemäß strömt der Liquiddampf dabei in einem Liquidströmungskanal 9 von der Liquidheizvorrichtung 4 zur Ansaugöffnungsanordnung 8 und der Tabakdampf strömt in einem Tabakströmungskanal 10 von dem Kammerinnenraum 6a zur Ansaugöffnungsanordnung 8. Durch ein Einatmen bzw. Ziehen an der Ansaugöffnungsanordnung 8 kann der Benutzer den Liquiddampf und den Tabakdampf einatmen. Gemäß der Darstellung der Fig. 1, 2, 3 und 4 kann die Ansaugöffnungsanordnung 8 eine einzelne Ansaugöffnung 8a aufweisen.

Die vorschlagsgemäße Rauchvorrichtung ist nun dadurch gekennzeichnet, dass der Liquidströmungskanal 9 in dem Gehäuse 1 außerhalb des Kammerinnenraums 6a verläuft. Mit anderen Worten vermeidet der Liquidströmungskanal 9 den Innenraum der Heizkammer 6 und führt insbesondere nicht durch den Kammerinnenraum 6a hindurch. Vielmehr besteht ein Abstand zwischen dem Liquidströmungskanal 9 und dem Kammerinnenraum 6a. Dabei kann der Liquidströmungskanal 9 allerdings prinzipiell außen entlang an der Heizkammer 6 verlaufen, solange der Kammerinnenraum 6a nicht durchquert wird. Insbesondere kann auch der Liquidverdampfer 2 hinsichtlich des Tabakströmungskanals 10 stromabwärts angeordnet sein.

In diesem Zusammenhang und gemäß einer ersten bevorzugten Variante wird dieser Verlauf des Liquidströmungskanals 9 außerhalb des Kammerinnenraums 6a dadurch erreicht, dass der Tabakströmungskanal 10 durch die Liquidheizvorrichtung 4 hindurchführt. Mit anderen Worten führt der Tabakströmungskanal 10 durch die Liquidheizvorrichtung 4 hindurch, wie insbesondere in den Fig. 1, 5 und 6 für diese Ausführungsbeispiele zu erkennen ist.

Ab der Liquidheizvorrichtung 4 fallen gemäß der Fig. 1, 5 und 6 erkennbar der Tabakströmungskanal 10 und der Liquidströmungskanal 9 zusammen. Dementsprechend ist es bevorzugt, dass stromabwärts von der Liquidheizvorrichtung 4, wobei sich diese Richtungsangabe sowohl auf das Strömen des Liquiddampfes als auch auf das Strömen des Tabakdampfes bezieht, der Tabakströmungskanal 10 dem Liquidströmungskanal 9 entspricht. Also verläuft auch hier erkennbar der Liquidströmungskanal 9 außerhalb des Kammerinnenraums 6, da der Liquidströmungskanal 9 in Strömungsrichtung beabstandet zu der Heizkammer 6 ist.

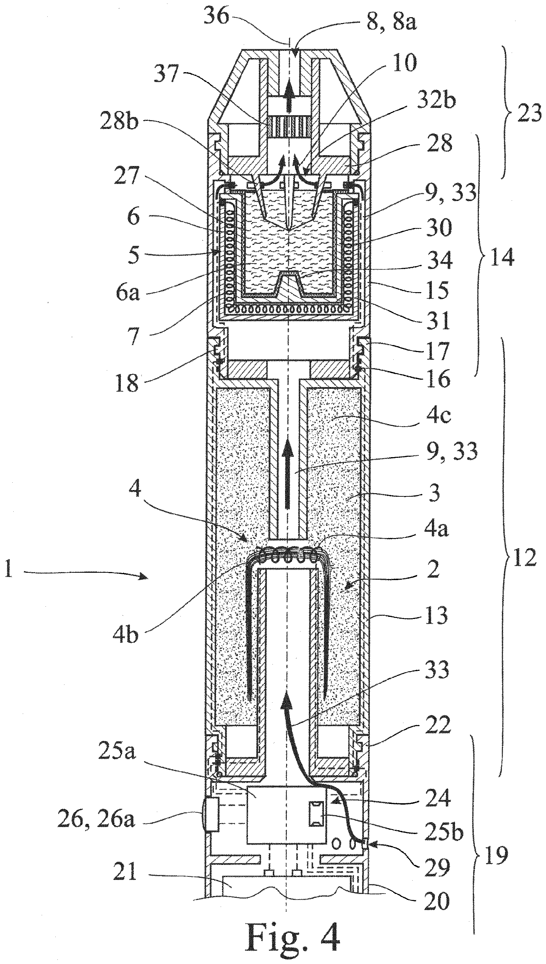

Alternativ ist vorgesehen, wie in der Fig. 4 gemäß dem zweiten Ausführungsbeispiel dargestellt ist, dass der Liquidströmungskanal 9 mit dem Tabakströmungskanal 10 an einem Kanalzusammenfluss 11 zusammengeführt wird, wobei der Kanalzusammenfluss 11 im Tabakströmungskanal 10 stromabwärts von dem Kammerinnenraum 6a angeordnet ist. Die Definition von stromabwärts entspricht hier wiederum der obigen Definition. Der Liquidströmungskanal 9 und der Tabakströmungskanal 10 vereinen sich also erst außerhalb der Heizkammer 6 und somit auch außerhalb des Kammerinnenraums 6a, sodass im Wesentlichen kein Liquiddampf in die Heizkammer 6 eintritt.

Der Liquidverdampfer 2 und der Tabakerhitzer 5 können auf einzelne Gehäuseteile verteilt sein, was für alle Ausführungsbeispiele in den Figuren dargestellt ist. So ist vorzugsweise vorgesehen, dass die Rauchvorrichtung ein Liquidverdampfermodul 12 mit einem Liquidverdampfergehäuseteil 13 aufweist, wobei der Liquidverdampfer 2 in dem Liquidverdampfergehäuseteil 13 angeordnet ist. Gemäß dieser bevorzugten Ausgestaltung weist die Rauchvorrichtung auch ein Tabakerhitzermodul 14 mit einem Tabakerhitzergehäuseteil 15 auf, wobei der Tabakerhitzer 5 in dem Tabakerhitzergehäuseteil 15 angeordnet ist.

Weiter ist bevorzugt, dass das Liquidverdampfermodul 12 lösbar mit dem Tabakerhitzermodul 14 durch eine Modulverbindung verbunden ist. Bei dieser Modulverbindung kann bevorzugt vorgesehen sein, dass sie eine mechanische und eine elektrische Verbindung zwischen dem Verdampfermodul 12 und dem Tabakwärmmodul 14 bereitstellt. In beiden Ausführungsbeispielen sind entsprechende Modulkontakte 16 für eine solche elektrische Verbindung dargestellt.

Zum Herstellen dieser Modulverbindung ist bevorzugt vorgesehen, dass das Liquidverdampfermodul 12 eine Verdampferverbindungsanordnung 17 und das Tabakerhitzermodul 14 eine Erhitzerverbindungsanordnung 18 jeweils zum Herstellen der Modulverbindung aufweist. Vorzugsweise handelt es sich, wie in den Fig. 1, 4, 5 und 6 dargestellt, bei der Modulverbindung um eine Bajonettverbindung, also um eine Verbindung nach der Art eines Bajonettverschlusses. Entsprechend kann die Verdampferverbindungsanordnung 17 und die Erhitzerverbindungsanordnung 18 jeweils Bajonettverbindungsmittel aufweisen, z. B. entsprechende Bajonettstifte oder Bajonettschlitze.

Wie bereits beschrieben, speist sich sowohl die Kammerheizvorrichtung 7 als auch die Liquidheizvorrichtung 4 bevorzugt aus elektrischer Energie. Daher ist es bevorzugt, dass die Rauchvorrichtung ein Akkumodul 19 mit einem Akkugehäuseteil 20 und einer in dem Akkugehäuseteil 20 angeordneten elektrischen Stromquelle 21 umfasst. Diese elektrische Stromquelle 21 ist zum Betreiben der Kammerheizvorrichtung 7 und der Liquidheizvorrichtung 4 eingerichtet. Bei der elektrischen Stromquelle 21 kann es sich sowohl um eine Batterie als auch um einen wieder aufladbaren Akku handeln, z. B. auf Lithiumionenbasis. Zum Aufladen der elektrischen Stromquelle kann die Rauchvorrichtung an dem Gehäuse 1 angeordnete und hier nicht gezeigte Ladekontakte für ein elektrisches Aufladen der elektrischen Stromquelle aufweisen.

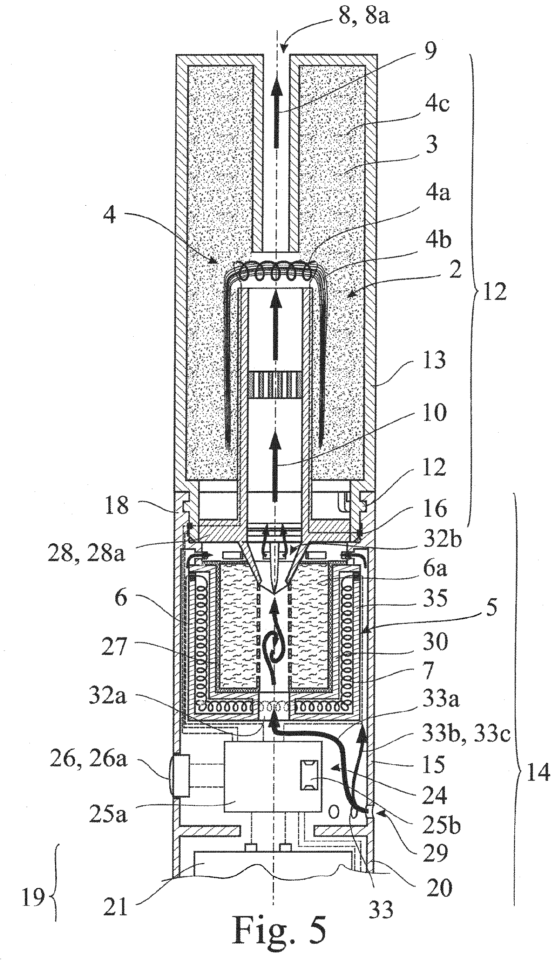

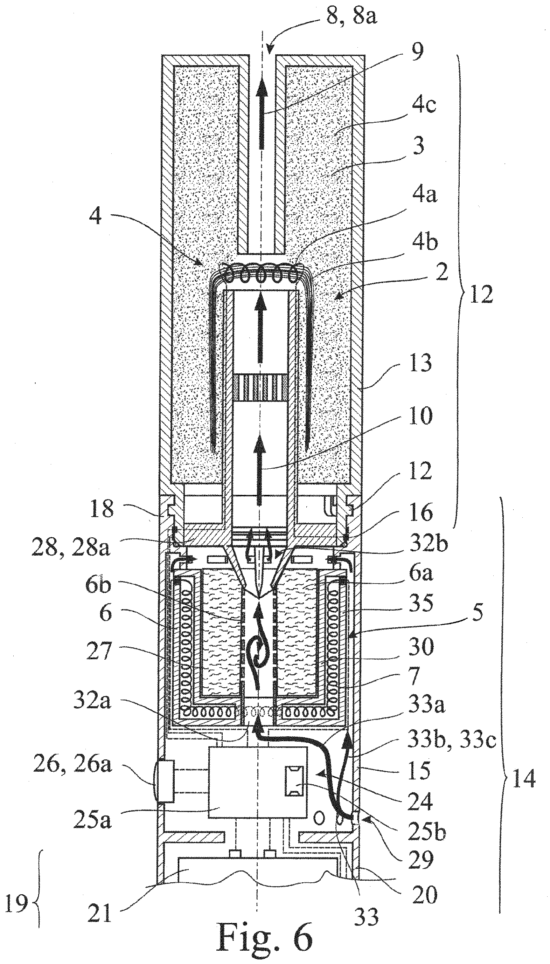

Nach einer Möglichkeit ist das Akkumodul 19 und speziell der Akkugehäuseteil 20 einstückig mit einem der anderen Module oder Gehäuseteile ausgebildet. So ist im ersten Ausführungsbeispiel gemäß der Fig. 1, im dritten Ausführungsbeispiel gemäß der Fig. 5 und im vierten Ausführungsbeispiel gemäß der Fig. 6 das Akkumodul 19 und damit auch das Akkugehäuseteil 20 einstückig mit dem Tabakerhitzermodul 14 und dem Tabakerhitzergehäuseteil 15 gestaltet.

Andererseits kann gemäß einer bevorzugten Variante und entsprechend dem zweiten Ausführungsbeispiel gemäß der Fig. 4 auch vorgesehen sein, dass das Akkumodul 19 eine Akkuverbindungsanordnung 22 zum Herstellen einer Akkuverbindung aufweist. Diese Akkuverbindung kann ebenfalls eine Bajonettverbindung sein. Entsprechend kann die Akkuverbindungsanordnung 22 auch Bajonettverbindungsmittel umfassen, was ebenfalls in der Fig. 4 dargestellt ist. Weiter kann insbesondere vorgesehen sein, dass die Akkuverbindung eine mechanische und elektrische Verbindung mit dem Tabakerhitzermodul 14 und/oder mit dem Liquidverdampfermodul 12 ist. Im Ausfuhrungsbeispiel der Fig. 4 besteht eine solche Verbindung zwischen dem Akkumodul 19 und dem Liquidverdampfermodul 12.

Es kann sich bei der obigen Akkuverbindung um eine Bajonettverbindung handeln, welche identisch zu der obigen Modulverbindung zwischen dem Liquidverdampfermodul 12 und dem Tabakerhitzermodul 14 ist. Dann kann die Akkuverbindungsanordnung 22 mit der Verdampferverbindungsanordnung 17 und der Erhitzerverbindungsanordnung 18 gleichermaßen eine Bajonettverbindung herstellen. Bezogen auf das Ausführungsbeispiel der Fig. 4 könnte auf diese Weise etwa die Position des Tabakerhitzermoduls 14 mit derjenigen des Liquidverdampfermoduls 12 - unter Beibehaltung der Funktionalität der Rauchvorrichtung insgesamt - ausgetauscht werden.

Eine entsprechende identische Verbindung, welche auch eine Bajonettverbindung ist, kann auch zwischen dem Tabakerhitzermodul 14 - wie in der Fig. 4 gezeigt - oder alternativ mit dem Liquidverdampfermodul 12 einerseits mit einem separaten Mundstückmodul 23 lösbar und damit flexibel hergestellt werden. Es könnte auch das Tabakerhitzermodul 14 oder das Liquidverdampfermodul 12 durch ein hinsichtlich seines Gehäuses im Wesentlichen identisches Dummymodul - welches nicht dargestellt ist - ersetzt werden, welches auch identisch gestaltete Verbindungsanordnungen aufweist, aber lediglich eine Durchführung von elektrischen Leitungen und Strömungskanälen bietet.

Auf diese Weise kann die Rauchvorrichtung dynamisch so konfiguriert werden, dass entweder nur die Funktionalität des verbliebenen Tabakerhitzermoduls 14 oder des verbliebenen Liquidverdampfermoduls 12 bestehen bleibt. Auf diese Weise können auch ein oder mehrere weitere Funktionsmodule hinzugefügt und etwa zwischen dem Liquidverdampfermodul 12 und dem Tabakerhitzermodul 14 - ausgehend von der Ausfuhrungsform der Fig. 4 - angeordnet werden.

Regelmäßig weist eine Rauchvorrichtung im Sinne des Vorschlags eine Steueranordnung 24 auf, welche in den hier gezeigten Beispielen eine Platine 25a und elektronische Bausteine 25b umfasst, die auf der Platine 25a angeordnet sind. Eine solche Steueranordnung 24 schaltet etwa die von der elektrischen Stromquelle 21 bereitgestellte Spannung an die Liquidheizvorrichtung 4 oder an die Kammerheizvorrichtung 7 durch. Daher ist es bevorzugt, dass die Rauchvorrichtung eine Steueranordnung 24 zur Ansteuerung der Kammerheizvorrichtung 7 und vorzugsweise auch der Liquidheizvorrichtung 4 umfasst, wobei die Ansteuerung der Kammerheizvorrichtung 7 zum wahlweisen Erhitzen der Heizkammer 6 auf eine Vorheiztemperatur oder auf eine Verfahrenstemperatur dient. Bei der Vorheiztemperatur entsteht im Wesentlichen kein Tabakdampf aus der Tabaksubstanz, aber es kann verhältnismäßig schnell auf die Verfahrenstemperatur, bei der dieser Tabakdampf entsteht, weiter erhitzt werden. Die genaue Wahl der Vorheiztemperatur und der Verfahrenstemperatur kann von der vorgesehenen Tabaksubstanz abhängig sein, z. B. davon, ob loser Tabak oder ein mit einem Präparat getränkter Tabak zur Verwendung im Tabakerhitzer 5 vorgesehen ist. Ein beispielhafter Wert für die Vorheiztemperatur beträgt 150° Celsius, wobei dann die Verfahrenstemperatur bei 165° Celsius liegen kann. Denkbar sind aber auch höhere Verfahrenstemperaturen, so etwa zwischen 180° Celsius und 200° Celsius oder sogar bis etwa 300° Celsius. Bei höheren Verfahrenstemperaturen kann auch eine zumindest teilweise Verbrennung der Tabaksubstanz auftreten.

Vorzugsweise ist die Verfahrenstemperatur einstellbar, sodass beispielsweise auch für unterschiedliche Tabakarten oder Zusatzsubstanzen ein unterschiedlicher Raucheffekt erreicht werden kann. Dazu weist die Steueranordnung 24 vorzugsweise eine Eingabevorrichtung 26 auf zum Einstellen der Verfahrenstemperatur. Bei dieser Eingabevorrichtung 26 kann es sich, wie in den Ausführungsbeispielen dargestellt, um eine Tastvorrichtung 26a handeln. Durch Betätigung dieser Tastvorrichtung 26a kann zwischen verschiedenen, z. B. voreingestellten und auswählbaren, Verfahrenstemperaturen gewechselt werden. Die aktuell gültige Auswahl kann dann beispielsweise durch eine - hier nicht dargestellte - LED-Anordnung optisch wiedergegeben werden. Denkbar wäre auch ein Drehring als Eingabevorrichtung 26. Die Eingabevorrichtung 26 kann auch zum Einschalten der Rauchvorrichtung und insbesondere zum Auslösen eines Vorheizens der Heizkammer 6 auf die Vorheiztemperatur dienen.

Über die Steueranordnung 24 kann auch eine Zugluftsteuerung sowohl der Liquidheizvorrichtung 4 als auch der Kammerheizvorrichtung 7 erfolgen. Wenn die Steueranordnung 24 über eine entsprechende Sensorik ein Einatmen bzw. "Ziehen" des Benutzers an der Ansaugöffnungsanordnung 8 wahrnimmt, kann die Liquidheizvorrichtung 4 so bestromt bzw. die Kammerheizvorrichtung 7 so angesteuert werden, dass die Verfahrenstemperatur zum Erzeugen des Tabakdampfes erreicht wird. Hier sind auch komplexere Steuerungen denkbar, wo etwa auf ein gepulstes Einatmen auf bestimmte Art und Weise reagiert wird. Denkbar wäre es auch, über eine solche Zugluftsteuerung ein Vorheizen auf die Vorheiztemperatur zu starten oder die Verfahrenstemperatur einzustellen.

Ein Aspekt einer solchen Rauchvorrichtung ist das Befüllen der Heizkammer 6, welche z. B. mit losem Tabak - wie in der Fig. 6 dargestellt - oder mit speziellen Tabakpads 27 - wie jeweils in den Fig. 1, 3, 4 und 5 dargestellt - erfolgen kann, wobei diese Tabakpads 27 entweder natürlichen oder präparierten Tabak in einer verschlossenen Kapsel umfassen. Solche Tabakpads 27 werden auch als "Pellets" bezeichnet. Hierfür ist bevorzugt vorgesehen, dass die Rauchvorrichtung einen öffenbaren Befülldeckel 28 zum Abdecken der Heizkammer 6 aufweist, wobei im geöffneten Zustand des Befülldeckels 28 der Kammerinnenraum 6a mit der Tabaksubstanz befüllt werden kann. Dafür kommt hier, wie bereits festgestellt, sowohl loser Tabak als auch ein Tabakpad 27 infrage.

Weiter ist bevorzugt, dass wie in dem jeweiligen Ausführungsbeispiel der Fig. 1, 5 und 6 dargestellt, der Befülldeckel 28 durch eine Deckelanordnung 28a des Liquidverdampfermoduls 12 gebildet wird. In diesem Ausführungsbeispiel ist diese Deckelanordnung 28a auch ein Bestandteil der Verdampferverbindungsanordnung 17. Dies führt zu der bevorzugten Variante, gemäß der durch ein Lösen der Modulverbindung der Befülldeckel 28 durch eine Trennung - hier des Liquidverdampfermoduls 12 - von der Heizkammer 6 geöffnet wird. Die lösbare Modulverbindung zwischen dem Liquidverdampfermodul 12 und dem Tabakerhitzermodul 14, welche bereits für einen Austausch des Liquidverdampfermoduls 12, z. B. wegen Verbrauch des Liquids, benötigt wird, kann somit auch für einen Zugang zu der Heizkammer 6 verwendet werden. Damit entfällt die Notwendigkeit, einen separaten Befülldeckel 28 mit einem entsprechenden Öffnungsmechanismus vorsehen zu müssen. Der Befülldeckel 28 kann - wie für die Ausführungsbeispiele dargestellt - einen Kapseldorn 28b zum Durchstechen des Tabakpads 27 aufweisen.

Hinsichtlich eines Lufteintrittes für die Luft, die dann aus der Ansaugöffnungsanordnung 8 herausgesaugt werden kann, ist bevorzugt vorgesehen, dass die Rauchvorrichtung eine Einlassöffnungsanordnung 29 aufweist, sodass ein eintretender Luftstrom durch den Liquidströmungskanal 9 und/oder durch den Tabakströmungskanal 10 zur Ansaugöffnungsanordnung 8 strömt. Diese Einlassöffnungsanordnung 29 kann einerseits, wie in dem Ausführungsbeispiel der Fig. 1, 5 und 6, in dem Tabakerhitzergehäuseteil 15 angeordnet sein. Ebenso kann die Einlassöffnungsanordnung 29 in dem Akkugehäuseteil 20 angeordnet sein, was im Ausführungsbeispiel der Fig. 4 gezeigt wird.

In diesem Zusammenhang ist bevorzugt weiter vorgesehen, dass ein Einlassströmungskanal 33 für den eintretenden Luftstrom von der Einlassöffnungsanordnung 29 zu der Heizkammer verläuft. Insbesondere kann es sein, dass der Einlassströmungskanal 33 zu einer Kammereinlassöffnung 32a der Heizkammer 6 verläuft, wobei die Kammereinlassöffnung 32a zum Einströmen des eintretenden Luftstroms eingerichtet ist, sodass der eintretende Luftstrom zur Aufnahme des Tabakdampfs die Heizkammer 6 zumindest teilweise quert. Es kann insbesondere sein, dass der Einlassströmungskanal 33 sich in einen ersten Einlassteilkanal 33a und in einen zweiten Einlassteilkanal 33b aufteilt, wobei dann - gemäß der Darstellung in der Fig. 5 und in der Fig. 6 - nur der erste Einlassteilkanal 33a zu der Kammereinlassöffnung 32a der Heizkammer 6 verläuft und nur der entsprechende Teil des eintretenden Luftstroms zur Aufnahme des Tabakdampfs die Heizkammer 6 wiederum zumindest teilweise quert.

Bezüglich der Heizkammer 6 ist bevorzugt vorgesehen, dass die Heizkammer 6 zylinderartig ist und eine geschlossene Mantelfläche 30 aufweist. Die Heizkammer 6 selbst kann auch eine geschlossene Grundfläche 31 - wie in dem zweiten Ausführungsbeispiel gemäß der Fig. 4 dargestellt - aufweisen, wobei alternativ hier auch eine zumindest teilweise offene Grundfläche infrage kommt, was speziell das dritte und das vierte Ausführungsbeispiel jeweils gemäß der Fig. 5 und der Fig. 6 betrifft, da bei diesen die teilweise offene Grundfläche 31 durch die Kammereinlassöffnung 32a der Heizkammer 6 herrührt.

Weiter ist bevorzugt, dass die Heizkammer 6 eine Kammerauslassöffnung 32b zum Ausströmen von Tabakdampf aufweist. Diese Kammerauslassöffnung 32b kann, wie für alle Ausführungsbeispiele dargestellt, durch eine teilweise oder - wie hier -vollständig offene und damit fehlende Dachfläche der Heizkammer 6 gebildet werden. Bezogen auf die Einlassöffnungsanordnung 29 kann auch vorgesehen sein, dass der Einlassströmungskanal 33 von der Einlassöffnungsanordnung 29 zur Kammerauslassöffnung 32b verläuft, was hier in den ersten beiden Ausführungsbeispielen gemäß der Fig. 1 und 4 dargestellt ist. Alternativ kann aber auch vorgesehen sein, dass ein Umgehungsströmungskanal 33c von der Einlassöffnungsanordnung 29 zur Kammerauslassöffnung 32b verläuft. Dieser Umgehungsströmungskanal 33c kann alternativ oder zusätzlich zu einem Strömungskanal - hier der erste Einlassteilkanal 33a - vorhanden sein, welcher von der Einlassöffnungsanordnung 29 zu einer Kammereinlassöffnung 32a der Heizkammer 6 verläuft. Bei den Ausführungsbeispielen der Fig. 5 und 6 entspricht dieser Umgehungsströmungskanal 33c jeweils dem zweiten Einlassteilkanal 33b.

Bevorzugt ist auch, dass die Kammereinlassöffnung 32a in einer Grundfläche 31 der Heizkammer 6 und da speziell gegenüberliegend zur Kammerauslassöffnung 32b angeordnet ist. Es können die Kammereinlassöffnung 32a und die Kammerauslassöffnung 32b auf einer Längsachse 36 des Gehäuses 1 angeordnet sein. Diese Merkmale sind ebenfalls in den Fig. 5 und 6 für das dritte und das vierte Ausführungsbeispiel dargestellt.

Speziell in dem Fall, dass zur Aufnahme des Tabakdampfs der eintretende Luftstrom die Heizkammer 6 zumindest teilweise quert, kann eine wirksame Trennung der festen Tabaksubstanz von dem eintretenden Luftstrom gewünscht sein, damit lediglich der Tabakdampf mitgeführt wird und alle festen Bestandteile der Tabaksubstanz hingegen in dem Kammerinnenraum 6a verbleiben. Daher ist es bevorzugt, dass die Heizkammer 6 eine luftdurchlässige Separieranordnung 6b zum Trennen der Tabaksubstanz aufweist, sodass der eintretende Luftstrom die Heizkammer 6 durch die Separieranordnung 6 von der Tabaksubstanz getrennt quert. Die Separieranordnung 6b kann dabei, wie speziell in dem vierten Ausführungsbeispiel der Fig. 6 gezeigt, rohrartig geformt und mit porenartigen Öffnungen versehen sein. Dabei sind die porenartigen Öffnungen vorzugsweise so ausgeformt, dass sie für den Tabakdampf durchlässig sind, die Tabaksubstanz im Übrigen aber zuverlässig zurückhalten. Das dritte Ausführungsbeispiel der Fig. 5 hingegen zeigt eine Variante, bei der der Tabakpad 27 eine Hülle aufweist, welche selbst an den betreffenden Abschnitten diese Luftdurchlässigkeit und gleichzeitig die Undurchlässigkeit für die Tabaksubstanz im Übrigen aufweist.

Wie ebenfalls in dem vierten Ausführungsbeispiel der Fig. 6 dargestellt ist es ferner bevorzugt, dass die Separieranordnung 6 zum schließenden Eingriff mit dem Befülldeckel 28 eingerichtet ist. Da bevorzugt der eintretende Luftstrom mit dem Verlassen der Heizkammer 6 gemäß diesem Ausführungsbeispiel bevorzugt durch den Befülldeckel 28 treten und in das Liquidverdampfermodul 12 strömen soll, kann auf diese Weise die - im geöffneten Zustand des Befülldeckels 28 - vorhandene Öffnung der Separieranordnung 6 geschlossen werden. Bevorzugt erfolgt dieser schließende Eingriff mit der Deckelanordnung 28a, also bevorzugt einem Bestandteil der Verdampferverbindungsanordnung 17. Wie ebenfalls in der Fig. 6 gezeigt kann es sich dabei um den, hier in den Kammerinnenraum 6a ragenden, Kapseldorn 28b handeln.

Bei einer einfach zylinderartig geformten Heizkammer 6 erfolgt die Erhitzung der Tabaksubstanz ausschließlich durch die Mantelfläche 30 und ggf. die Grundfläche 31 sowie einer etwaig vorhandenen Dachfläche. Um die wirksame Oberfläche zur Erhitzung zu vergrößern, kann weiter vorgesehen sein, dass die Heizkammer 6 einen durch die Kammerheizvorrichtung 7 erhitzbaren zylinder- oder kegelartigen Innenvorsprung 34 aufweist, wie für die ersten beiden Ausführungsbeispiele in den Fig. 1, 3 und 4 dargestellt ist.

Die Temperaturzunahme, welche mit dem Erhitzen der Heizkammer 6 einhergeht, ist regelmäßig weder an der Liquidheizvorrichtung 4, noch an dem Gehäuse 1 erwünscht. Um eine Temperaturzunahme an diesen Stellen zu minimieren, ist daher bevorzugt vorgesehen, dass der Tabakerhitzer 5 eine Thermalbarriere 35 zur thermischen Isolierung der Heizkammer 6 aufweist. Um auch eine Reinigung der Heizkammer 6 und speziell des Kammerinnenraums 6a von Rückständen der Tabaksubstanz zu erlauben, weist die Heizkammer 6 vorzugsweise eine Keramikinnenbeschichtung auf.

Eine besonders ergonomische Form sowie eine vorteilhafte Modularität ergibt sich für die Rauchvorrichtung, wenn - wie für beide Ausführungsbeispiele dargestellt - das Gehäuse 1 im Wesentlichen zylinderartig geschaltet ist und der Liquidverdampfer 2 sowie der Tabakerhitzer 5 auf der Längsachse 36 des Gehäuses 1 angeordnet sind. Vorzugsweise gilt diese Anordnung auf der Längsachse 36 auch für das Liquidverdampfermodul 12 sowie alternativ oder zusätzlich auch für das Tabakerhitzermodul 14.

Die dargestellten Ausführungsbeispiele unterscheiden sich wie bereits beschrieben, hinsichtlich der Reihenfolge der Anordnung des Liquidverdampfers 2 und des Tabakerhitzers 5 in Bezug auf die Strömungsrichtung. Die Ausführungsbeispiele der Fig. 1 und der Fig. 5 und 6 sehen nun vor, dass die Ansaugöffnungsanordnung 8 an einem Mundstückende 35 der Rauchvorrichtung angeordnet ist und dass bezogen auf die Längsachse 36 der Liquidverdampfer 2, vorzugsweise auch das Verdampfermodul 12, mundstückseitig zum Tabakerhitzer 5 und damit vorzugsweise auch mundstückseitig zu dem Tabakerhitzermodul 14 angeordnet ist. Mit anderen Worten ist nicht nur hinsichtlich der Strömung, sondern auch geometrisch der Liquidverdampfer 2 zwischen dem Mundstückende 35 und dem Tabakerhitzer 5 platziert. Im Gegensatz dazu sieht die Ausführungsform der Fig. 4 vor, dass bezogen auf die Längsachse 36 der Tabakerhitzer 5 und vorzugsweise auch das Tabakerhitzermodul 14 mundstückseitig zum Liquidverdampfer 2 und vorzugsweise auch zum Liquidverdampfermodul 12 angeordnet ist.

Gerade für die Varianten der Fig. 1 sowie 5 und 6, bei denen der Tabakdampf durch die Liquidheizvorrichtung 4 geführt ist, kann eine Verschmutzung der Liquidheizvorrichtung 4 durch diesen Tabakdampf auftreten. Daher ist es bevorzugt, dass die Rauchvorrichtung und insbesondere der Liquidverdampfer 2 eine Filteranordnung 37, bei dem es sich vorzugsweise um einen Aktivkohlefilter handelt, im Tabakströmungskanal 10 umfasst und hier kann speziell diese Filteranordnung 37 zwischen der Heizkammer 6 und der Liquidheizvorrichtung 4 im Tabakströmungskanal 10 angeordnet sein, was auch aus der Darstellung der Fig. 1 sowie 5 und 6 hervorgeht.

## Patentansprüche

1. Rauchvorrichtung mit einem Gehäuse (1), einem in dem Gehäuse (1) angeordneten Liquidverdampfer (2), welcher ein Reservoir (3) für Liquid und eine Liquidheizvorrichtung (4) zum Verdampfen des Liquid in Liquiddampf aufweist, sowie einem in dem Gehäuse (1) angeordneten Tabakerhitzer (5), welcher eine Heizkammer (6) zur Aufnahme einer Tabaksubstanz in einem Kammerinnenraum (6a) und eine Kammerheizvorrichtung (7) zum Erzeugen von Tabakdampf aus der Tabaksubstanz aufweist, und einer Ansaugöffnungsanordnung (8) zum Ansaugen des Liquiddampfes und zum Ansaugen des Tabakdampfs aus dem Gehäuse (1), wobei der Liquiddampf in einem Liquidströmungskanal (9) von der Liquidheizvorrichtung (4) zur Ansaugöffnungsanordnung (8) strömt und der Tabakdampf in einem Tabakströmungskanal (10) von dem Kammerinnenraum (6a) zur Ansaugöffnungsanordnung (8) strömt,
**dadurch gekennzeichnet, dass**
der Liquidströmungskanal (9) in dem Gehäuse (1) außerhalb des Kammerinnenraums (6a) verläuft.

2. Rauchvorrichtung nach Anspruch 1, **dadurch gekennzeichnet, dass** der Tabakströmungskanal (10) durch die Liquidheizvorrichtung (4) hindurchführt, vorzugsweise, dass stromabwärts von der Liquidheizvorrichtung (4) der Tabakströmungskanal (10) dem Liquidströmungskanal (9) entspricht.

3. Rauchvorrichtung nach Anspruch 1, **dadurch gekennzeichnet, dass** der Liquidströmungskanal (9) mit dem Tabakströmungskanal (10) an einem Kanalzusammenfluss (11) zusammengeführt wird, wobei der Kanalzusammenfluss (11) im Tabakströmungskanal (10) stromabwärts von dem Kammerinnenraum (6a) angeordnet ist.

4. Rauchvorrichtung nach einem der Ansprüche 1 bis 3, **dadurch gekennzeichnet, dass** die Rauchvorrichtung ein Liquidverdampfermodul (12) mit einem Liquidverdampfergehäuseteil (13) aufweist, wobei der Liquidverdampfer (2) in dem Liquidverdampfergehäuseteil (13) angeordnet ist, und ein Tabakerhitzermodul (14) mit einem Tabakerhitzergehäuseteil aufweist (15), wobei der Tabakerhitzer (5) in dem Tabakerhitzergehäuseteil (15) angeordnet ist, vorzugsweise, dass das Liquidverdampfermodul (12) lösbar mit dem Tabakerhitzermodul (14) durch eine Modulverbindung verbunden ist, insbesondere, dass die Modulverbindung eine mechanische und eine elektrische Verbindung zwischen dem Liquidverdampfermodul (12) und dem Tabakerhitzermodul (14) bereitstellt.

5. Rauchvorrichtung nach Anspruch 4, **dadurch gekennzeichnet, dass** das Liquidverdampfermodul (12) eine Verdampferverbindungsanordnung (17) und das Tabakerhitzermodul (14) eine Erhitzerverbindungsanordnung (18), jeweils zum Herstellen der Modulverbindung aufweist, vorzugsweise, dass die Modulverbindung eine Bajonettverbindung ist.

6. Rauchvorrichtung nach einem der Ansprüche 1 bis 5, **dadurch gekennzeichnet, dass** die Rauchvorrichtung ein Akkumodul (19) mit einem Akkugehäuseteil (20) und einer in dem Akkugehäuseteil (20) angeordneten elektrischen Stromquelle (21) zum Betreiben der Liquidheizvorrichtung (4) und der Kammerheizvorrichtung (7) aufweist, vorzugsweise, dass die Rauchvorrichtung an dem Gehäuse (1) angeordnete Ladekontakte für ein elektrisches Aufladen der elektrischen Stromquelle (21) aufweist.

7. Rauchvorrichtung nach Anspruch 6, **dadurch gekennzeichnet, dass** das Akkumodul (19) eine Akkuverbindungsanordnung (22) aufweist zum Herstellen einer Akkuverbindung, vorzugsweise, welche Akkuverbindung eine Bajonettverbindung ist, insbesondere, dass die Akkuverbindung eine mechanische und elektrische Verbindung mit dem Tabakerhitzermodul (14) und/oder mit dem Liquidverdampfermodul (12) ist.

8. Rauchvorrichtung nach einem der Ansprüche 1 bis 7, **dadurch gekennzeichnet, dass** die Rauchvorrichtung eine Steueranordnung (24) zur Ansteuerung der Kammerheizvorrichtung (7), vorzugsweise auch der Liquidheizvorrichtung (4), umfasst, zum wahlweisen Erhitzen der Heizkammer (6) auf eine Vorwärmtemperatur oder auf eine, vorzugsweise einstellbare, Verfahrenstemperatur, insbesondere, dass die Steueranordnung (24) eine Eingabevorrichtung (26), vorzugsweise eine Tastvorrichtung (26a), zum Einstellen der Verfahrenstemperatur aufweist.

9. Rauchvorrichtung nach einem der Ansprüche 1 bis 8, **dadurch gekennzeichnet, dass** die Rauchvorrichtung einen öffenbaren Befülldeckel (28) zum Abdecken der Heizkammer (6) aufweist, wobei im geöffneten Zustand des Befülldeckels (28) der Kammerinnenraum (6a) mit Tabaksubstanz befüllt werden kann, vorzugsweise, dass der Befülldeckel (28) durch eine Deckelanordnung (28a) des Liquidverdampfermoduls (12) gebildet wird, insbesondere, dass durch ein Lösen der Modulverbindung der Befülldeckel (28) durch eine Trennung von der Heizkammer (6) geöffnet wird.

10. Rauchvorrichtung nach einem der Ansprüche 1 bis 9, **dadurch gekennzeichnet, dass** die Rauchvorrichtung eine Einlassöffnungsanordnung (29) aufweist, sodass ein eintretender Luftstrom durch den Liquidströmungskanal (9) und/oder durch den Tabakströmungskanal (10) zur Ansaugöffnungsanordnung (8) strömt.

11. Rauchvorrichtung nach Anspruch 10, **dadurch gekennzeichnet, dass** ein Einlassströmungskanal (33) für den eintretenden Luftstrom von der Einlassöffnungsanordnung (29) zu der Heizkammer (6) verläuft, insbesondere, zu einer Kammereinlassöffnung (32a) der Heizkammer (6) zum Einströmen des eintretenden Luftstroms verläuft, sodass der eintretende Luftstrom zur Aufnahme des Tabakdampfs die Heizkammer (6) zumindest teilweise quert.

12. Rauchvorrichtung nach einem der Ansprüche 1 bis 11, **dadurch gekennzeichnet, dass** die Heizkammer (6) zylinderartig ist und eine geschlossene Mantelfläche (30) aufweist, insbesondere eine geschlossene Grundfläche (31) aufweist.

13. Rauchvorrichtung nach einem der Ansprüche 1 bis 12, **dadurch gekennzeichnet, dass** die Heizkammer (6) eine Kammerauslassöffnung (32b) zum Ausströmen von Tabakdampf aufweist, vorzugsweise, dass die Kammereinlassöffnung (32a) in einer Grundfläche (31) der Heizkammer (6), insbesondere gegenüberliegend zur Kammerauslassöffnung (32b), angeordnet ist, weiter vorzugsweise, dass die Kammereinlassöffnung (32a) und die Kammerauslassöffnung (32b) auf einer Längsachse (36) des Gehäuses (1) angeordnet sind.

14. Rauchvorrichtung nach Anspruch 13, **dadurch gekennzeichnet, dass** die Heizkammer (6) eine luftdurchlässige Separieranordnung (6b) zum Trennen der Tabaksubstanz aufweist, sodass der eintretende Luftstrom die Heizkammer (6) durch die Separieranordnung (6) von der Tabaksubstanz getrennt quert.

15. Rauchvorrichtung nach Anspruch 14, **dadurch gekennzeichnet, dass** die Separieranordnung (6) zum schließenden Eingriff mit dem Befülldeckel (28), vorzugsweise mit der Deckelanordnung (28a) und insbesondere mit einem in den Kammerinnenraum (6a) ragenden Kapseldorn (28b), eingerichtet ist.

16. Rauchvorrichtung nach einem der Ansprüche 1 bis 15, **dadurch gekennzeichnet, dass** ein Umgehungsströmungskanal (33c) von der Einlassöffnungsanordnung (29) zur Kammerauslassöffnung (32b) verläuft, insbesondere, dass die Heizkammer (6) einen durch die Kammerheizvorrichtung (7) erhitzbaren zylinder- oder kegelartigen Innenvorsprung (34) aufweist.

17. Rauchvorrichtung nach Anspruch 16, **dadurch gekennzeichnet, dass** die Ansaugöffhungsanordnung (28) an einem Mundstückende (35) der Rauchvorrichtung angeordnet ist und dass, bezogen auf die Längsachse (36), der Liquidverdampfer (2), vorzugsweise das Liquidverdampfermodul (12), mundstückseitig zum Tabakerhitzer (5), vorzugsweise zu dem Tabakerhitzermodul (14), angeordnet ist.

18. Rauchvorrichtung nach Anspruch 16, **dadurch gekennzeichnet, dass** der Umgehungsströmungskanal (33c) zusätzlich zu einem Strömungskanal, weiter vorzugsweise zu einem Einlassteilkanal (33a), vorhanden ist, welcher von der Einlassöffnungsanordnung (29) zu einer Kammereinlassöffnung (32a) der Heizkammer (6) verläuft.

19. Rauchvorrichtung nach einem der Ansprüche 1 bis 18, **dadurch gekennzeichnet, dass** ein Einlassströmungskanal (33) für den eintretenden Luftstrom von der Einlassöffnungsanordnung (29) zu der Heizkammer (6) verläuft, vorzugsweise, dass der Einlassströmungskanal (33) sich in einen ersten Einlassteilkanal (33a) und in einen zweiten Einlassteilkanal (33b) aufteilt, wobei nur der erste Einlassteilkanal (33a) zu der Kammereinlassöffnung (32a) der Heizkammer (6) verläuft und nur der entsprechende Teil des eintretenden Luftstroms zur Aufnahme des Tabakdampfs die Heizkammer (6) zumindest teilweise quert.

## Claims

1. Smoking device having a housing (1), having a liquid evaporator (2), which is arranged in the housing (1) and has a reservoir (3) for liquid and also a liquid-heating device (4) for evaporating the liquid into liquid vapor, and having a tobacco heater (5), which is arranged in the housing (1) and has a heating chamber (6) for accommodating a tobacco substance in a chamber interior (6a) and also a chamber-heating device (7) for generating tobacco vapor from the tobacco substance, and having an intake-opening arrangement (8) for taking in the liquid vapor, and for taking in the tobacco vapor, from the housing (1), wherein the liquid vapor flows in a liquid-flow channel (9) from the liquid-heating device (4) to the intake-opening arrangement (8) and the tobacco vapor flows in a tobacco-flow channel (10) from the chamber interior (6a) to the intake-opening arrangement (8),
**characterized in that**
the liquid-flow channel (9) runs in the housing (1), outside the chamber interior (6a).

2. Smoking device according to Claim 1, **characterized in that** the tobacco-flow channel (10) passes through the liquid-heating device (4), preferably **in that**, downstream of the liquid-heating device (4), the tobacco-flow channel (10) corresponds to the liquid-flow channel (9).

3. Smoking device according to Claim 1, **characterized in that** the liquid-flow channel (9) is guided together with the tobacco-flow channel (10) at a channel junction (11), wherein the channel junction (11) is arranged in the tobacco-flow channel (10), downstream of the chamber interior (6a).

4. Smoking device according to one of Claims 1 to 3, **characterized in that** the smoking device has a liquid-evaporator module (12) with a liquid-evaporator housing part (13), wherein the liquid evaporator (2) is arranged in the liquid-evaporator housing part (13), and has a tobacco-heater module (14) with a tobacco-heater housing part (15), wherein the tobacco heater (5) is arranged in the tobacco-heater housing part (15), preferably **in that** the liquid-evaporator module (12) is connected to the tobacco-heater module (14) in a releasable manner by a module connection, in particular that the module connection establishes a mechanical and an electrical connection between the liquid-evaporator module (12) and the tobacco-heater module (14).

5. Smoking device according to Claim 4, **characterized in that** the liquid-evaporator module (12) has an evaporator-connecting arrangement (17) and the tobacco-heater module (14) has a heater-connecting arrangement (18), each for establishing the module connection, preferably **in that** the module connection is a bayonet connection.

6. Smoking device according to one of Claims 1 to 5, **characterized in that** the smoking device has a storage-battery module (19) with a storage-battery housing part (20) and an electric power source (21), arranged in the storage-battery housing part (20), for operating the liquid-heating device (4) and the chamber-heating device (7), preferably in that the smoking device has charging contacts which are arranged on the housing (1) and are intended for electrically charging up the electric power source (21).

7. Smoking device according to Claim 6, **characterized in that** the storage-battery module (19) has a storage-battery connecting arrangement (22) for establishing a storage-battery connection, said storage-battery connection preferably being a bayonet connection, in particular **in that** the storage-battery connection is a mechanical and electrical connection to the tobacco-heater module (14) and/or to the liquid-evaporator module (12).

8. Smoking device according to one of Claims 1 to 7, **characterized in that** the smoking device comprises a control arrangement (24) for activating the chamber-heating device (7), preferably also the liquid-heating device (4), for optionally heating the heating chamber (6) to a pre-heating temperature or to a, preferably adjustable, process temperature, in particular **in that** the control arrangement (24) has an input device (26), preferably a touch-contact device (26a), for adjusting the process temperature.

9. Smoking device according to one of Claims 1 to 8, **characterized in that** the smoking device has an openable filling cap (28) for covering the heating chamber (6), wherein, in the open state of the filling cap (28), the chamber interior (6a) can be filled with tobacco substance, preferably **in that** the filling cap (28) is formed by a cap arrangement (28a) of the liquid-evaporator module (12), in particular **in that**, by virtue of the module connection being released, the filling cap (29) is opened by being separated from the heating chamber (6).

10. Smoking device according to one of Claims 1 to 9, **characterized in that** the smoking device has an inlet-opening arrangement (29), in which case an entering air stream flows through the liquid-flow channel (9) and/or through the tobacco-flow channel (10) to the intake-opening arrangement (8).

11. Smoking device according to Claim 10, **characterized in that** an inlet-flow channel (33) for the entering air stream runs from the inlet-opening arrangement (29) to the heating chamber (6), in particular to a chamber-inlet opening (32a) of the heating chamber (6) for the inward flow of the entering air stream, such that the entering air stream for taking up the tobacco vapor at least partially crosses the heating chamber (6).

12. Smoking device according to one of Claims 1 to 11, **characterized in that** the heating chamber (6) is cylindrical and has a closed lateral surface (30), in particular has a closed base surface (31).

13. Smoking device according to one of Claims 1 to 12, **characterized in that** the heating chamber (6) has a chamber outlet opening (32b) for the outward flow of tobacco vapor, preferably **in that** the chamber inlet opening (32a) is arranged in a base surface (31) of the heating chamber (6), especially opposite the chamber outlet opening (32b), and more preferably **in that** the chamber inlet opening (32a) and the chamber outlet opening (32b) are arranged on a longitudinal axis (36) of the housing (1).

14. Smoking device according to Claim 13, **characterized in that** the heating chamber (6) has an air-permeable separating arrangement (6b) for separation of the tobacco substance, such that the entering air stream crosses the heating chamber (6) separated from the tobacco substance by the separating arrangement (6).

15. Smoking device according to Claim 14, **characterized in that** the separating arrangement (6) is designed for closure engagement with the filling cap (28), preferably with the cap arrangement (28a) and in particular with a capsule spike (28b) which protrudes into the chamber interior (6a).

16. Smoking device according to one of Claims 1 to 15, **characterized in that** a bypass-flow channel (33c) runs from the inlet-opening arrangement (29) to the chamber-outlet opening (32b), in particular **in that** the heating chamber (6) has a cylindrical or conical inner protrusion (34) which can be heated by the chamber-heating device (7).

17. Smoking device according to Claim 16, **characterized in that** the intake-opening arrangement (28) is arranged at a mouthpiece end (35) of the smoking device, and **in that**, as seen in relation to the longitudinal axis (36), the liquid evaporator (2), preferably the liquid evaporator module (12), is arranged on the mouthpiece side in relation to the tobacco heater (5), preferably in relation to the tobacco-heater module (14).

18. Smoking device according to Claim 16, **characterized in that** a bypass-flow channel (33c) is present in addition to a flow channel, more preferably in addition to an inlet sub-channel (33a), which runs from the inlet-opening arrangement (29) to a chamber inlet opening (32a) of the heating chamber (6).

19. Smoking device according to one of Claims 1 to 18, **characterized in that** an inlet-flow channel (33) for the entering air flow runs from the inlet-opening arrangement (29) to the heating chamber (6), preferably **in that** the inlet-flow channel (3) divides into a first inlet sub- channel (33a) and a second inlet sub-channel (33b), wherein only the first inlet sub-channel (33a) leads to the chamber inlet opening (32a) of the heating chamber (6), and only the corresponding portion of the entering air stream at least partially crosses the heating chamber (6) in order to take up the tobacco vapor.

## Revendications

1. Dispositif destiné à fumer présentant un boîtier (1), un évaporateur de liquide (2) agencé dans le boîtier (1), lequel présente un réservoir (3) pour liquide et un dispositif destiné à chauffer le liquide (4) destiné à vaporiser le liquide en une vapeur (1), ainsi qu'un système de chauffage du tabac (5) agencé dans le boîtier (1), lequel présente une chambre de chauffage (6) destinée à accueillir une substance de tabac dans un compartiment interne de la chambre (6a) et un dispositif de chauffage de la chambre (7) destiné à produire de la vapeur de tabac à partir de la substance de tabac, et un agencement d'orifice d'aspiration (8) destiné à aspirer la vapeur et à aspirer la vapeur du tabac à partir du boîtier (1), la vapeur s'écoulant au sein d'un canal d'écoulement du liquide (9) depuis le dispositif destiné à chauffer le liquide (4) vers l'agencement d'orifice d'aspiration (8) et la vapeur de tabac s'écoulant dans un canal d'écoulement du tabac (10) depuis le compartiment interne de la chambre (6a) vers l'agencement d'orifice d'aspiration (8),
**caractérisé en ce que**
le canal d'écoulement liquide (9) dans le boîtier (1) s'étend en dehors du compartiment interne de la chambre (6a).

2. Dispositif destiné à fumer selon la revendication 1, **caractérisé en ce que** le canal d'écoulement du tabac (10) passe à travers le dispositif destiné à chauffer le liquide (4), de préférence **en ce que** le canal d'écoulement du tabac (10) correspond, en aval du dispositif destiné à chauffer le liquide (4), au canal d'écoulement du liquide (9).

3. Dispositif destiné à fumer selon la revendication 1, **caractérisé en ce que** le canal d'écoulement du liquide (9) se confond avec le canal d'écoulement du tabac (10) au niveau d'une jonction du canal (11), la jonction du canal (11) étant agencée dans le canal d'écoulement du tabac (10) en aval du compartiment interne de la chambre (6a).

4. Dispositif destiné à fumer selon l'une quelconque des revendications 1 à 3, **caractérisé en ce que** le dispositif destiné à fumer présente un module formant évaporateur de liquide (12) avec une partie boîtier de l'évaporateur de liquide (13), l'évaporateur de liquide (2) étant agencé dans la partie boîtier de l'évaporateur de liquide (13) et un module du système de chauffage du tabac (14) présentant une partie boîtier du système de chauffage du tabac (15), dans lequel le système de chauffage (5) est agencé dans la partie boîtier du système de chauffage du tabac (15), de préférence, **en ce que** le module formant évaporateur de liquide (12) est relié de manière amovible au module du système de chauffage du tabac (14) par une liaison modulaire, en particulier, **en ce que** la liaison modulaire fournit une liaison mécanique et électrique entre le module formant évaporateur de liquide (12) et le module du système de chauffage du tabac (14).

5. Dispositif destiné à fumer selon la revendication 4, **caractérisé en ce que** le module formant évaporateur de liquide (12) présente un agencement de liaisons d'évaporateur et le module du système de chauffage du tabac (14) un agencement de liaison de système de chauffe (18), respectivement destiné à la génération de la liaison modulaire, de préférence **en ce que** la liaison modulaire est une liaison à baïonnette.

6. Dispositif destiné à fumer selon l'une quelconque des revendications 1 à 5, **caractérisé en ce que** le dispositif destiné à fumer présente un module de batterie (19) avec une partie boîtier de batterie (20) et une source (21) de courant électrique agencée dans la partie boîtier de batterie (20) destinée à actionner le dispositif destiné à chauffer le liquide (4) et le dispositif de chauffage de la chambre (7), de préférence, **en ce que** le dispositif destiné à fumée présente des contacts de charge agencés au niveau du boîtier (1) pour une charge électrique de la source de courant électrique (21).

7. Dispositif destiné à fumer selon la revendication 6, **caractérisé en ce que** le module de batterie (19) présente un agencement de liaison de batterie (22) destiné à la génération d'une liaison de la batterie, de préférence laquelle liaison de batterie est une liaison à baïonnette, notamment, **en ce que** la liaison de batterie est une liaison mécanique et électrique avec le module du système de chauffage du tabac (14) et/ou avec le module d'évaporation de liquide (12).

8. Dispositif destiné à fumer selon l'une quelconque des revendications 1 à 7, **caractérisé en ce que** le dispositif destiné à fumer comprend un agencement de commande (24) destiné à entraîner le dispositif de chauffage de la chambre (7), de préférence également le dispositif destiné à chauffer le liquide (4) pour chauffer sélectivement la chambre de chauffage (6) à une température de préchauffage ou à une température de traitement de préférence réglable, en particulier **en ce que** l'agencement de la commande (24) présente un dispositif d'entrée (26), de préférence un dispositif tactile (26a), pour régler la température de traitement.

9. Dispositif destiné à fumer selon l'une quelconque des revendications 1 à 8, **caractérisé en ce que** le dispositif destiné à fumer présente un couvercle de remplissage ouvrable (28) destiné à couvrir la chambre de chauffage (6), le compartiment interne de la chambre (6a) pouvant être rempli avec la substance de tabac à l'état ouvert du couvercle de remplissage (28) de préférence, **en ce que** le couvercle de remplissage (28) est formé par un agencement de couvercle (28a) du module d'évaporation de liquide (12), en particulier, **en ce que**, via un relâchement de la liaison modulaire, le couvercle de remplissage est ouvert par une séparation de la chambre de chauffage (6).

10. Dispositif destiné à fumer selon l'une quelconque des revendications 1 à 9, **caractérisé en ce que** le dispositif destiné à fumer présente un agencement d'ouverture d'admission (29) de telle sorte qu'un courant d'air s'écoule à travers le canal d'écoulement du liquide (9) et/ou le canal d'écoulement du tabac (10) vers l'agencement d'orifice d'aspiration (8).

11. Dispositif destiné à fumer selon la revendication 10, **caractérisé en ce qu'**un canal d'écoulement d'admission (33) pour le courant d'air entrant s'étend de l'agencement d'ouverture d'admission (29) à la chambre de chauffage (6), et en particulier, s'étend à une ouverture d'admission de la chambre (32a) de la chambre de chauffage (6) pour permettre l'afflux du courant d'air entrant de sorte que le courant d'air entrant traverse au moins partiellement la chambre de chauffage (6) pour absorber la vapeur de tabac.

12. Dispositif destiné à fumer selon l'une quelconque des revendications 1 à 11, **caractérisé en ce que** la chambre de chauffage (6) est de forme cylindrique et présente une surface enveloppante fermée (30), en particulier **en ce qu'**elle présente une surface de base fermée (31).

13. Dispositif destiné à fumer selon l'une quelconque des revendications 1 à 12, **caractérisé en ce que** la chambre de chauffage (6) présente une ouverture d'admission de la chambre (32b) destinée à dégager de la vapeur de tabac de préférence **en ce que** l'ouverture d'admission de la chambre (32a) est agencée dans une surface de base (31) de la chambre de chauffage (6), en particulier de manière opposée à l'ouverture d'admission de la chambre (32b), de préférence, **en ce que** l'ouverture d'admission de la chambre (32a) et l'ouverture d'admission de la chambre (32b) sont agencées sur un axe longitudinal (36) du boîtier (1).

14. Dispositif destiné à fumer selon la revendication 13, **caractérisé en ce que** la chambre de chauffage (6), présente un agencement de séparation perméable à l'air (6b) pour séparer la substance de tabac de sorte que le courant d'air entrant traverse, de manière séparée de la substance de tabac, la chambre de chauffage (6) par l'agencement de séparation (6).

15. Dispositif destiné à fumer selon la revendication 14, **caractérisé en ce que** l'agencement de séparation (6) est aménagé pour un engagement de fermeture avec le couvercle de remplissage (28), de préférence avec l'agencement de couvercle (28a), et notamment avec une pointe de capsule saillante à l'intérieur de la chambre (6a).

16. Dispositif destiné à fumer selon l'une quelconque des revendications 1 à 15, **caractérisé en ce qu'**un canal d'écoulement de dérivation (33c) s'étend de l'agencement d'ouverture d'admission (29) à l'ouverture d'admission de la chambre (32b), notamment **en ce que** la chambre de chauffage (6) présente une saillie intérieure (34) en forme cylindrique ou en forme de cône chauffable via le dispositif de chauffage de la chambre (7).

17. Dispositif destiné à fumer selon la revendication 16, **caractérisé en ce que** l'agencement d'orifice d'aspiration (28) est agencé à une extrémité de l'embouchure (35) du dispositif destiné à fumer, et que, par rapport à l'axe longitudinal (36), l'évaporateur de liquide (2), de préférence le module formant évaporateur de liquide (12) est agencé côté pièce d'embouchure vers le système de chauffage du tabac (5), de préférence vers le module du système de chauffage du tabac (14).

18. Dispositif destiné à fumer selon la revendication 16, **caractérisé en ce que** le canal d'écoulement de dérivation (33c) existe, en plus d'un canal d'écoulement, plus préférablement d'un canal partie d'entrée (33a), lequel s'étend de l'agencement d'ouverture d'admission (29) à l'ouverture d'admission de la chambre (32a) de la chambre de chauffage (6).

19. Dispositif destiné à fumer selon l'une quelconque des revendications 1 à 18, **caractérisé en ce qu'**un canal d'écoulement d'admission (33) pour le courant d'air entrant s'étend de l'agencement d'ouverture d'admission (29) à la chambre de chauffage (6), de préférence **en ce que** le canal d'écoulement d'admission (33) se divise en un premier canal partie d'admission (33a) et en un deuxième canal partie d'admission (33b), le premier canal partie d'admission (33a) s'étendant de l'ouverture d'admission de la chambre (32a) à la chambre de chauffage (6) et **en ce que** seule la partie correspondante du courant d'air entrant traverse au moins partiellement la chambre de chauffage (6) pour absorber la vapeur de tabac.
